# EUROPEAN PATENT APPLICATION

(11) **EP 4 631 423 A1**
(43) Date of publication of application: **15.10.2025**
(21) Application number: 25169625.8
(22) Date of filing: 10.04.2025
(51) Int. Cl.: A61B 5/0205, A61B 5/024, A61B 5/08, A61B 5/00

(54) **MONITORING APPARATUS**

(30) Priority: 11.04.2024 FI 20245454
(71) Applicant: Monidor Oy, 90590 Oulu (FI)
(72) Inventor: SAVOLA, Mikko, 90590 Oulu (FI); PUOLITAIVAL, Antti, 90590 Oulu (FI); PUOLITAIVAL, Seppo, 90590 Oulu (FI); PUURUNEN, Veli-Matti, 90590 Oulu (FI); NISSILÄ, Seppo, 90590 Oulu (FI)
(74) Representative: Kolster Oy Ab

(57) **Abstract**

An apparatus is disclosed. The invention relates to monitoring lung condition. The solution comprises non-invasive optical measurements (300) on a patient utilising at least two wavelengths; determining (302), based on the optical measurements, oxygen saturation, heart rate and heart rate variations of the patient; determining (304), based on heart rate and/or heart rate variations, breathing effort of the measurement subject; detecting (306) a change in breathing effort; detecting (308) a change in oxygen saturation level; determining (310), based the detected changes, a probability index for lung problem; comparing (312) the probability index to a given threshold and generating (314) an indication if the probability index is over a given threshold.

## Description

### Technical Field

The exemplary and non-limiting embodiments of the invention relate generally to apparatuses for non-invasive monitoring of oxygen saturation and pulse of an individual.

### Background

There are many medical conditions that are difficult detect because the symptoms are varied and sometimes unclear. Hydration problems are one of these conditions which may become apparent also in lungs such as a pulmonary edema, a medical condition marked by an abnormal accumulation of fluid in the lungs and which poses significant challenges to respiratory function and overall health. This condition manifests in two primary forms: cardiogenic and non-cardiogenic pulmonary edema.

Prompt medical intervention is vital to address many lung conditions such as pulmonary edema. Effective management not only alleviates symptoms but also mitigates the risk of complications, underscoring the importance of timely diagnosis and intervention in pulmonary edema cases. Thus, detecting pulmonary edema is of upmost importance. Further, it is also important to detect health conditions and condition changes which will lead or possibility lead to pulmonary edema, such as hydration status and changes, especially during or related to infusion care, breathing changes, oxygen saturation changes, heart rate or heart rate variability changes.

### Brief description

According to an aspect of the present invention, there is provided an apparatus for monitoring lung condition, the apparatus comprising at least one sensor; at least one processor; at least one memory including computer program code; the at least one memory and the computer program code configured to, with the at least one processor, cause the apparatus at least to perform: perform, utilising the at least one sensor, non-invasive optical measurements on a measurement subject utilising at least two wavelengths; determine, based on the optical measurements, oxygen saturation, heart rate and/or heart rate variations of the measurement subject; determine, based on heart rate and/or heart rate variations, breathing effort of the measurement subject; detect a change in breathing effort; detect a change in oxygen saturation level; determine, based on the detected changes, a probability index for lung problem; compare the probability index to a given threshold; generate an indication if the probability index is over a given threshold.

According to an aspect of the present invention, there is provided a method for monitoring lung condition, the method comprising performing, utilising at least one sensor, non-invasive optical measurements on a measurement subject utilising at least two wavelengths; determining, based on the optical measurements, oxygen saturation, heart rate and/or heart rate variations of the measurement subject; determining, based on heart rate and/or heart rate variations, breathing effort of the measurement subject; detecting a change in breathing effort; detecting a change in oxygen saturation level; determining, based on the detected changes, a probability index for lung problem; comparing the probability index to a given threshold; generating an indication if the probability index is over a given threshold.

According to an aspect of the present invention, there is provided a computer program comprising instructions for causing an apparatus execute the steps of controlling a sensor to perform, utilising at least one sensor, non-invasive optical measurements on a measurement subject utilising at least two wavelengths; determining, based on the optical measurements, oxygen saturation, heart rate and/or heart rate variations of the measurement subject; determining, based on heart rate and/or heart rate variations, breathing effort of the measurement subject; detecting a change in breathing effort; detecting a change in oxygen saturation level; determining, based on the detected changes, a probability index for lung problem; comparing the probability index to a given threshold; generating an indication if the probability index is over a given threshold.

In an embodiment, the invention relates to an apparatus comprising means for performing, utilising at least one sensor, non-invasive optical measurements on a measurement subject utilising at least two wavelengths, means for determining, based on the optical measurements, oxygen saturation, heart rate and/or heart rate variations of the measurement subject, means for determining, based on heart rate and/or heart rate variations, breathing effort of the measurement subject, means for detecting a change in breathing effort, means for detecting a change in oxygen saturation level, means for determining, based on the detected changes, a probability index for lung problem,; means for comparing the probability index to a given threshold; and means for generating an indication if the probability index is over a given threshold.

One or more examples of implementations are set forth in more detail in the accompanying drawings and the description below. Other features will be apparent from the description and drawings, and from the claims. The embodiments and or examples and features, if any, described in this specification that do not fall under the scope of the independent claims are to be interpreted as examples useful for understanding various embodiments of the invention.

### Brief description of the drawings

In the following the invention will be described in greater detail by means of preferred embodiments with reference to the accompanying drawings, in which
Figures 1 and 2 illustrate an example of an apparatus of an embodiment;
Figure 3 is a flowchart illustrating an embodiment;
Figures 4 and 5 illustrate measurement examples of some embodiments;
Figures 6A, 6B, 6C, 6D, 6E and 6F illustrate applying thresholds;
Figures 7 and 8 illustrate an example of an apparatus of an embodiment;
Figure 9 illustrates a measurement example of an embodiment.

### Detailed description of some embodiments

As mentioned, there are many medical conditions that are difficult detect by the medical personnel due to the varied and sometimes undistinguishable symptoms. Undetected conditions may cause severe problems and cause danger to the wellbeing of the patients.

Let us study some examples.

Person A has cardiac dysfunction. He has been living at home without monitoring devices. His life is pretty normal, but he is not able to do heavy workouts. Suddenly he feels that his condition is weakening, and he gets exhausted even with light workouts.

He has a home health care device and he measures that his oxygen saturation is still high enough 92%, although it slightly decreased from his normal 95%.

As the condition is weakening the person goes to a hospital and gets a quick preliminary diagnosis of pneumonia and also a guess that he might have sepsis (blood poisoning). The blood pressure values are low 97/58 mmHg. Due to the latter one and low blood pressures he will get intravenous (IV)-infusion. During the next two hours he will receive 1000 ml NaCl 0,9% liquid. The blood pressure is monitored and the values are getting better to 123/72 mmHg. The infusion is continued with a Ringer-infusion, 500 ml in 45 minutes.

However, suddenly breathing becomes challenging, and heart rate goes up from 80bpm to 115 bpm. Breathing frequency goes up from to 22 breaths per minute. It is 3 hours since he came to the hospital.

A health care specialist recognizes the situation to be worse. She expects patient A has pneumonia. She measures the oxygen saturation, which is now 88%, and body temperature which is 39 °C. She decides to administer additional oxygen to patient A. The IV-infusion continues, 500 ml Ringer-liquid at a speed of 200 ml/hour. The patient becomes restless, and his breathing becomes more challenging. He uses all efforts to breathe using extra muscles and body, increases breathing frequency and intensity. However, the oxygen saturation continues to decrease to 85%. Patient's condition is worsening. Breathing frequency is now 28-32 breaths per minute. It is expected that the pulmonary edema has been developed.

The patient will be moved to the emergency room. The IV infusion has to be stopped although it has been helped at first to get blood pressure values to reasonable level.

In another example, a person B is a 72 year old man. He has been in pretty good condition, but suddenly feels a little pain in his chest. He can breathe normally. Body temperature is normal. Heart rate is a little bit elevated at 80bpm. Blood pressure values are low 118/72 mmHg. ECG measurement is almost normal, some indications from oxygen deficit.

More measurements and tests are done in a hospital. Blood haemoglobin is very low 62g/liter. Blood oxygen is however normal 98%. An action is taken to give him blood red cells. The plan is to give two bags. The first one is given in one hour. After that the heart rate is elevated from 80 bpm to 85, and then 90, 100 and 110 bpm measured in each 5 minutes respectively. Oxygen saturation is now 95%. The second blood package is going to be fed. However, the condition of the patient is worsening. The respiration of the patient is more difficult, he feels fewer. Breathing is becoming irregular and spontaneous needing more attention and extra power. Blood pressure goes up 145/92 mmHg. Blood saturation goes down 95%-91%-88%. For acute diagnosis X-ray image is going to be taken. It shows acute pulmonary edema.

The above examples emphasise that detecting respiratory conditions is important.

There are two medical issues which are related to each other and discussed here, hydration level and pulmonary edema.

Hydration problems in humans comprise a range of conditions, from mild dehydration or hypohydration to severe overhydration or hyperhydration, each posing distinct challenges to health and well-being.

Hypohydration and hyperhydration represent two contrasting states of the body's water balance, each with its own set of implications for health and well-being. Hypohydration, commonly known as dehydration, occurs when the body loses more fluids than it takes in, leading to an imbalance in fluid levels. This condition can arise from various factors such as excessive sweating during intense physical activity, inadequate fluid intake, haemorrhage or diseases.

In contrast, hyperhydration denotes an excess of water within the body, beyond its capacity for regulation or efficient excretion. It can result from excessive fluid intake, particularly when large amounts of water are consumed rapidly. Symptoms of hyperhydration include nausea, vomiting, confusion, and in severe instances, coma or death. Therefore, maintaining a proper balance of hydration is essential for optimal health, emphasizing the importance of monitoring fluid intake to prevent both hypohydration and hyperhydration.

Hypohydration may cause heart and internal organ problems and weaken the condition of a person. Hyperhydration in turn may be sign of heart failure or nonefficient heart function. In some disease cases, the accumulation of fluid such as pulmonary edema can develop quickly and deteriorate the heath condition of a patient in some hours.

There are various ways of monitoring hydration status of a patient or a person. The hydration status may be detected by a weight scale. The patient is weighed for example every morning/evening to follow up his/her weight and analyze if the hydration level is ok or not. However, this method is not accurate and fast changes cannot be detected. It can be also a challenge to ask or move a patient to a scale. In some cases when a patient cannot move or cannot be moved to a scale due to a health condition when a patient is lying on a bed. For example, a patient is going to get IV-infusion or he/she is getting IV-infusion or he/she has just got IV-infusion, and he/she is lying on a bed equipped with a monitoring device such as a blood oxygen monitor or blood pressure sensor or heart pulse monitor or ECG monitor, for example. Hydration status may be monitored with a skin impedance measurement. Further, the amount of water in the tissues can be measured by optical properties of the tissue. The tissues may be examiner optically by a non-invasive optical measurement where the wavelengths used can be selected to detect the absorption of water in the tissue. Skin impedance and optical skin water absorbance measurement can only measure local skin conditions and are not always accurate to estimate the whole body condition or internal body condition such as lung condition.

Pulmonary edema is a condition characterized by an abnormal accumulation of fluid in the lungs and surrounding tissues. This buildup of fluid disrupts the normal exchange of oxygen and carbon dioxide, resulting in breathing difficulties and potential health risks. Symptoms of pulmonary edema may include trouble breathing, coughing, the production of frothy or blood-tinged sputum, rapid heartbeat, anxiety, and bluish skin discoloration.

In general, pulmonary edema is detected through a combination of medical history, physical examination, and diagnostic tests.

The diagnostic tests may include X-ray imagining of the patient's chest, a computed tomography (CT) scan of the chest or various laboratory tests for blood samples. There is no single test for confirming that breathlessness is caused by pulmonary edema - there are many causes of shortness of breath; but currently there are no methods to suggest a high probability of an edema.

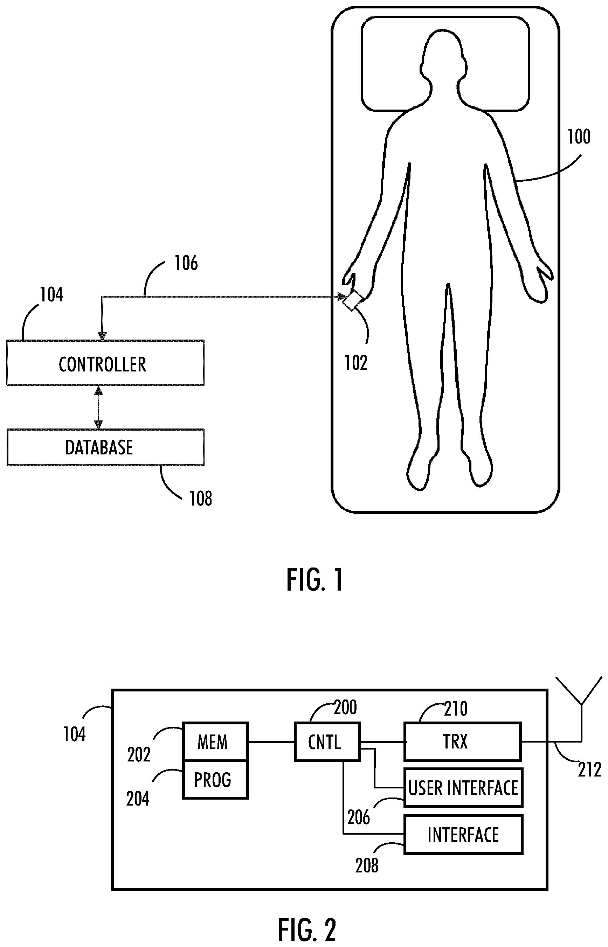

Figs. 1 and 2 illustrate an example of an apparatus for monitoring lung condition of a patient 100. The apparatus comprises a controller 104 operationally connected 106 to a sensor 102. The sensor is configured to perform non-invasive optical measurements on a patient utilising at least two wavelengths.

The controller 104 of the example includes a control circuitry 200 configured to control at least part of the operation of the apparatus. The control circuitry may be realized with at least one processor, for example.

The device may comprise a memory 202 for storing data. Furthermore, the memory may store software 204 executable by the control circuitry or processor 200. The memory may be integrated in the control circuitry.

The device may comprise a transceiver 210 which, in some embodiments, may be connected to an antenna 212.

The transceiver 210 may be a wireless transceiver utilizing known radio technology. An example of suitable radio technology is Bluetooth^{®}. Example of other possible technologies are wireless local area network (WLAN or WiFi), worldwide interoperability for microwave access (WiMAX), ZigBee^{®} and various cellular communication technologies well known in the art.

In an embodiment, the transceiver is a wired transceiver connected with a wired connection to a suitable network such as the Internet.

In an embodiment, the controller 104 is configured to communicate, utilising the transceiver 210, with a hospital database 108 and/or an external monitoring device, for example.

The device may comprise a user interface 206, which may comprise a display, a keypad and a speaker, for example. The display may be touch sensitive in which case a keypad or keyboard is not necessarily required. In an embodiment, the user interface is at least partly realized in a remote monitoring apparatus connected to the device via the transceiver in a wired or wireless manner.

In an embodiment, the sensor 102 is a pulse oximeter. A pulse oximeter is a sensor which measures the level of oxygen saturation in the blood without the need for invasive procedures. Oxygen saturation refers to the amount of oxygen dissolved in the blood, which is determined by detecting two forms of hemoglobin (Hb): oxyhemoglobin and deoxyhemoglobin. Hemoglobin is oxygenated, i.e., it contains oxygen. Deoxyhemoglobin is deoxygenated, i.e., it is hemoglobin that has released its oxygen to cells. The measurement of pulse oximeter is relying on the use of two distinct light wavelengths to gauge the absorption spectra of these hemoglobin types. For example, red light at approximately 660 nm and infrared light at approximately 940 nm are employed. The concentrations of oxygenated (HbO2) and deoxygenated (Hb) hemoglobin in the bloodstream affect the absorption of these wavelengths. Oxygenated hemoglobin absorbs more at 940 nm, while deoxygenated hemoglobin absorbs more at 660 nm. It is known that these two wavelengths can be selected from the absorption wavelength windows between 620-730 nm for oxygenated hemoglobin, and 810-960 nm for deoxygenated hemoglobin.

In an embodiment, transmissive pulse oximetry is employed, where a thin part of the patient's body, such as a fingertip or earlobe, is illuminated on one side while the photodetector is positioned on the opposite side. Fingertips and earlobes are usually chosen due to their relatively high blood flow, which helps maintain warmth, though this could be diminished in hypothermic patients. Alternatively, other viable sites include an infant's foot or the cheek or tongue of an unconscious patient.

In an embodiment, reflectance pulse oximetry is utilized, wherein both the illumination and the photodetector are placed on the same surface. This method doesn't necessitate a thin section of the body and can be applied to almost any part of the body. However, its accuracy may not be as reliable as the transmissive approach.

In an embodiment, a pulse oximeter comprises light emitting diodes that transmit two wavelengths. A photodetector in the oximeter detects the light not absorbed by the LEDs. Subsequently, this signal may undergo inversion through an inverting operational amplifier. The resulting signal delineates the light absorbed by the finger, which is then separated into a DC component and an AC component. The DC component signifies the absorption of light by the tissue, venous blood, and non-pulsatile arterial blood, while the AC component represents the pulsating blood. A pulse oximeter can also determine the heart rate and heart rate variation, pulse amplitude and its variation. Typically, an AC component of the signal is used for heart pulse detection. It is possible to use one wavelength or the both wavelengths of the pulse oximeter for heart pulse detection.

In an embodiment, also another or additional photoplethysmography (PPG) may be used in addition to is a pulse oximeter. In PPG, non-invasive optical measurements are used to detect volumetric changes in blood in peripheral circulation. A PPG sensor can measure heart pulses so it can measure heart rate and heart rate variation, pulse amplitude and its variations.

PPG utilizes low-intensity infrared (IR) light. As this light passes through biological tissues, it is absorbed by various components such as bones, skin pigments, and both venous and arterial blood. Due to the higher absorption of light by blood compared to surrounding tissues, PPG sensors can detect changes in blood flow by monitoring alterations in light intensity. The voltage signal obtained from PPG is directly proportional to the amount of blood moving through the blood vessels. This method can detect even minor fluctuations in blood volume, although it cannot precisely quantify the volume of blood. A PPG signal comprises multiple elements, such as a pulsatile (AC) waveform for synchronous cardiac changes in the blood volume with each heartbeat and is superimposed on a slowly varying (DC) baseline with several lower frequency components attributed to breathing, sympathetic nervous system activity, and thermoregulation. The accuracy of PPG recordings is influenced by factors such as the measurement site and the level of contact between the site and the sensor.

It is known that different PPG sensors are used in wearable head band, wrist device and other monitors. Typically, they use different wavelengths than used in pulse oximeters due to different use conditions such as during walking, running or other exercises. The selected wavelengths are used for motion artefact corrections and eliminations. One wavelength is typically close to 550 nm being green light, and another 650 nm being red or near infrared. A PPG sensor can use different wavelengths and it is not limited to the wavelengths mentioned above. In addition, the wavelengths used in a pulse oximeter can be used for detecting pulsatile blood flow and measure heart rate and heart rate variability.

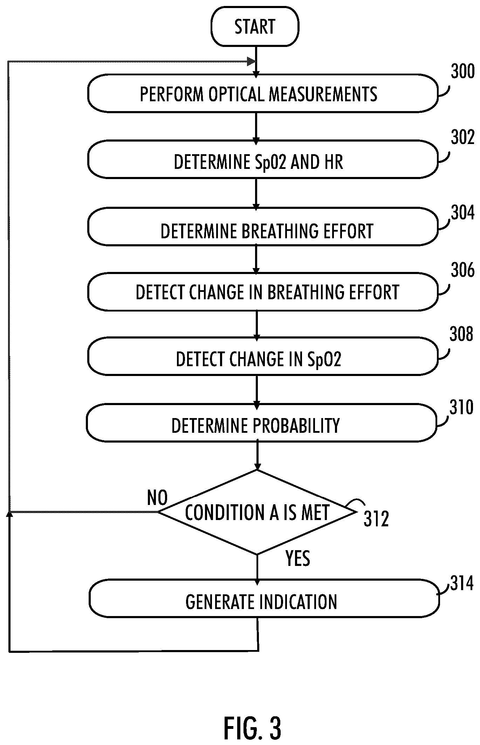

Fig. 3 is a flowchart illustrating some embodiments. Fig. 3 illustrates the operation of the apparatus of Figs. 1 and 2.

In step 300, the apparatus is configured to perform, utilising the at least one sensor 102, non-invasive optical measurements on a measurement subject or patient utilising at least two wavelengths.

In an embodiment, one of the at least two wavelengths is between 640 to 670 nm and another of the at least two wavelengths is between 910 to 940 nm. In an embodiment, the measurements are based on pulse oximeter and/or PPG sensors. In an embodiment, the apparatus is an oxygen saturation measurement apparatus.

In step 302, the apparatus is configured to determine, based on the optical measurements, oxygen saturation, heart rate and/or heart rate variations of the measurement subject.

The controller 104 may be configured to analyse the signal from the at least one sensor to make the determination. The controller may analyse from the sensor signal the AC and/or DC level, DC voltage peak values, voltage peak to peak and signal frequency, for example. As such, these methods are known in the art.

In step 304, the apparatus is configured to determine, based on heart rate and/or heart rate variations, breathing effort of the patient.

In an embodiment, the breathing effort comprises breathing frequency, breathing amplitude, breathing intensity, and/or breathing volume.

In step 306, the apparatus is configured to detect a change in breathing effort, based on measurements.

In step 308, the apparatus is configured to detect a change in oxygen saturation level.

The changes may be detected when the measurements are performed for a given time period, for example.

In step 310, the apparatus is configured to determine, based on the detected changes, a probability index for lung problem.

In step 312, the apparatus is configured to compare the probability index to a given threshold. The threshold may be a system parameter or adjustable by personnel using the apparatus.

In step 314, if the probability index is over a given threshold, the apparatus is configured to generate an indication.

The procedure of Fig.3 may be performed continuously as long as deemed necessary.

In an embodiment, the apparatus may receive information on age, sex, medication of the measurement subject and take received information into account when determining the probability index. The information may be received from the database 108, for example.

In an embodiment, the lung condition is pulmonary edema.

It is known to recognize the heart pulsing from a pulse oximeter/PPG signal due to the optical absorption or reflectance of pulsing blood. When heart pulse is detected, it is possible to determine heart rate; beats per minute and further heart rate variation. The heart rate variation is a measure of how much heart rate varies on a beat-to-beat basis. Breathing modulates the heart rate; inhalation increasing heart rate and exhalation decreasing it. It is thus possible to determine breathing frequency from the heart rate beat-to-beat variation. Analysing the beat-to-beat variation is also possible to determine the consistency or regularity of breathing frequency. Especially fast changes such as coughing, yawning, throbbing can be found as an irregular event in breathing pattern and beat-to-beat variation of the heart. The detected heart pulse amplitude also varies due to breathing and breathing deepness. The variation amplitude of the heart rate variation is also a result of breathing deepness.

Breathing also affects the pulse oximeter/PPG signal in other ways. Thoracic pressure, for example, as well amount of oxygen in blood varies according to breathing. These changes can be seen as alternating (AC) and offset level (DC) changes or their combination in the pulse oximeter/PPG signal. Using the AC and DC signal components it is possible to detect deepness of breathing and/or breathing frequency or just use the signal for improving the accuracy and reliability of detection of breathing parameters.

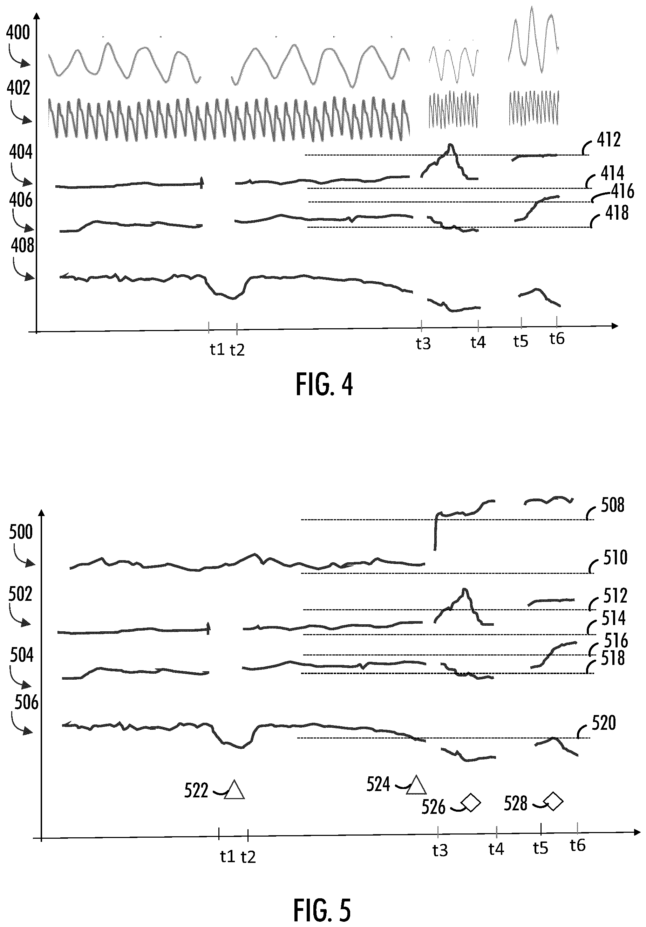

Fig. 4 illustrates an example of analysing the measurements. On x-axis is time and on y-axis are breathing (inhale-exhale) 400, pulse oximeter/PPG signal 402, breathing frequency 404, breathing amplitude 406 and oxygen saturation 408.

Before time instant t1, breathing 400 and heart rate 402 are normal. In an embodiment, these values may be used as a reference.

Between time instants t1-t2, breathing stops. Oxygen saturation 408 drops. Between time instants t2-t3, breathing is mostly normal, at the end of the period oxygen saturation starts to weaken, and breathing frequency 404 increases, which is a possible indication of a lung problem.

After time instant t3, a problem in breathing is detected. There are pauses, coughing or so, and after that during time instants t3-t4 oxygen saturation 408 drops, breathing frequency 404 increases, as well heart rate 402, breathing amplitude 406 decreases, meaning that there is probability for problematic lung condition.

Between time instants t5-t6, breathing frequency 404 is still high, breathing amplitude 406 is increased. The oxygen saturation 408 is increasing a little bit first but deteriorates despite of all breathing efforts, which again is a possible probability for problematic lung condition.

Fig 4 also shows example of possible thresholds. Thresholds 412 and 414 are breathing frequency 404 upper and lower thresholds, respectively. Thresholds 416 and 418 are breathing amplitude 406 upper and lower thresholds, respectively.

Fig. 5 illustrates another example of analysing the measurements. On x-axis is time and on y-axis are heart rate 500, breathing frequency 502, breathing amplitude 504 and oxygen saturation 506.

Fig 5 also shows example of possible thresholds. Thresholds 508 and 510 are heart rate 500 upper and lower thresholds, respectively. Thresholds 512 and 514 are breathing frequency 502 upper and lower thresholds, respectively. Thresholds 516 and 518 are breathing amplitude 504 upper and lower thresholds, respectively. Finally, threshold 520 is oxygen saturation 506 lower threshold.

Between time instants t1-t2 it is detected that oxygen saturation 506 drops under the lower limit 520. A probability index 522 is indicated, meaning that there is probability for problematic lung condition. Due to recovery at time t2, the probability index is reduced to nil. In an embodiment, the apparatus/controller can reduce the probability index over time when the condition is normal i.e. the signals are not exceeding the thresholds. For example, the probability index is reduced 1% per every minute when condition of the previous minute is detected as normal until the probability is zero.

At time instant t3, it is detected that oxygen saturation 506 again drops under the lower limit 520. A probability index 524 is indicated, meaning that there is probability for problematic lung condition.

Between time instants t3-t4 it is detected that one or more breathing parameters 502, 504 exceed limits 512, 516, also heart rate 500 is high, oxygen saturation 506 drops further. A higher probability index 526 is indicated, meaning that there is probability for problematic lung condition.

Between time instants t5-t6 it is detected that one or more breathing parameters 502, 504 again exceed limits 512, 516, also heart rate 500 is high, oxygen saturation 506 drops further. A higher probability index 528 is indicated. It is also indicated that there is high probability for problematic lung condition due to successive higher probability index indications.

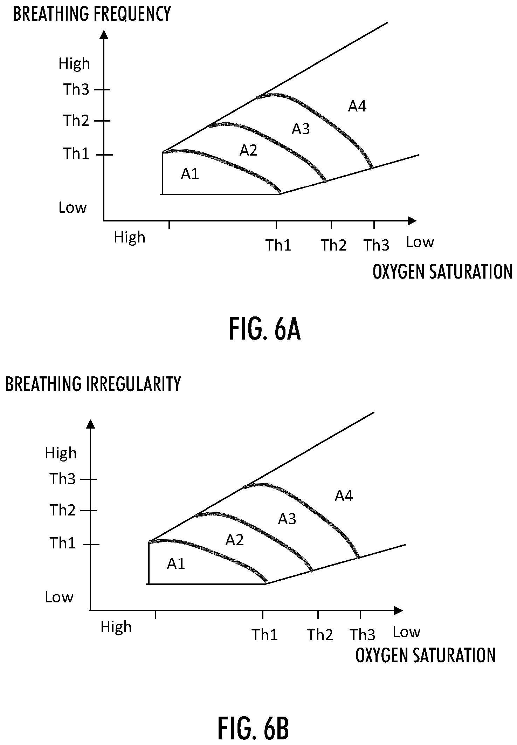

Figs. 6A to 6F describe examples how problematic lung conditions can be determined using different parameter combinations. The parameters (heart rate, oxygen saturation, breathing amplitude, breathing intensity, breathing amplitude, breathing irregularity) can be combined in many different ways. Each output/parameter can be defined using measurement data of oxygen saturation sensor or optical heart rate sensor such mentioned PPG sensor. The following Table 1 describes examples of measured signals and features, the determined feature from the measured signal or feature, and the related parameter defined from the determined feature.

**Table 1.**

| **Measured** | **Determined** | **Parameter** |
|---|---|---|
| HR pulse amplitude and/or time | Beat-to-beat variation in amplitude and/or time | Breathing frequency |
| HR pulse frequency, pulse | Pulse irregularity (missing pulses) | Breathing irregularity (missing breathing events) |
| HR pulse amplitude | Beat-to-beat amplitude variation | Breathing amplitude/intensity |
| Pulse AC signal | Beat-to-beat variation in amplitude and/or time | Breathing frequency |
| Pulse DC signal | Level and variation | Breathing amplitude/intensity |

In an embodiment, each parameter may be evaluated against a set of given one or more thresholds. In an embodiment, based on the values of the parameters I view of the thresholds, one or more probability factors may be determined. A probability index can then be calculated based on one or more probability factors. For example, if only one parameter is used, then the probability factor of the parameter may correspond directly to the probability index. If more than one parameter value is used, the probability index may be calculated based on the probability factors of the parameters.

Fig. 6A illustrates an example where on x-axis is oxygen saturation, and on y-axis the breathing frequency. If the breathing frequency (breathing effort) parameter is over a given threshold, a probability factor for the breathing frequency can be set respectively. For example, if the breathing frequency exceeds Th1 the probability factor can be set to 1 and if and breathing frequency exceeds Th2 the probability factor can be set to 2. Respectively, if the oxygen saturation level is under Th1 on x-axis the probability factor of the oxygen saturation level can be set 1, and if the oxygen saturation level is under Th2 on x-axis the probability factor can be set 2.

It is also possible to use two or more parameter combinations to evaluate the probability factor to the parameter pair.

In Figs 6A to 6F two selected parameters are compared to each other to evaluate the measured parameters and their abnormality and effect to the determine probability factor.

As an example, Figure 6A as mentioned illustrate oxygen saturation value on x-axis and breathing frequency on y-axis. If the measured parameter pair is in the area A1 i.e. oxygen saturation is higher than x-axis Th1 (note the direction of axis, higher values on left and lower values on right) and the breathing frequency is lower than y-axis Th1, then the value pair is NORMAL and the probability factor may be given the value of 0.

If the oxygen saturation-breathing frequency pair is in the area of A2, the value pair is abnormal. The probability factor may be given the value of 1. Similarly, if the oxygen saturation-breathing frequency pair is in the area of A3, the value pair is again abnormal and probability factor may be given the value of 2. Similarly, if the oxygen saturation-breathing frequency pair is in the area of A4, the value pair is again abnormal and probability factor may be given the value of 3.

The areas of Fig 6A are merely an example of possible division and classification of areas. The areas can be different sizes and having different values.

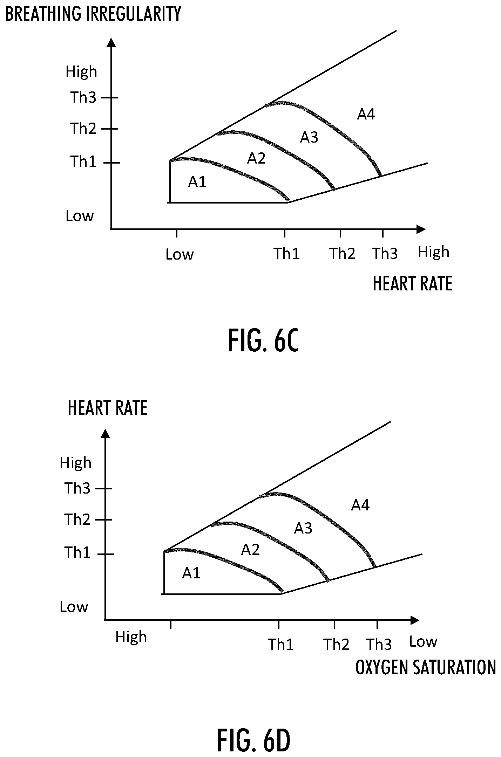

The system may use one or more parameter pairs to determine probability index. Fig 6B illustrates an example where parameter pairs are oxygen saturation level on x-axis and breathing irregularity on y-axis. Fig 6C illustrates an example where parameter pairs are heart rate on x-axis and breathing irregularity on y-axis.

Fig 6D illustrates an example where parameter pairs are oxygen saturation level on x-axis and heart rate on y-axis.

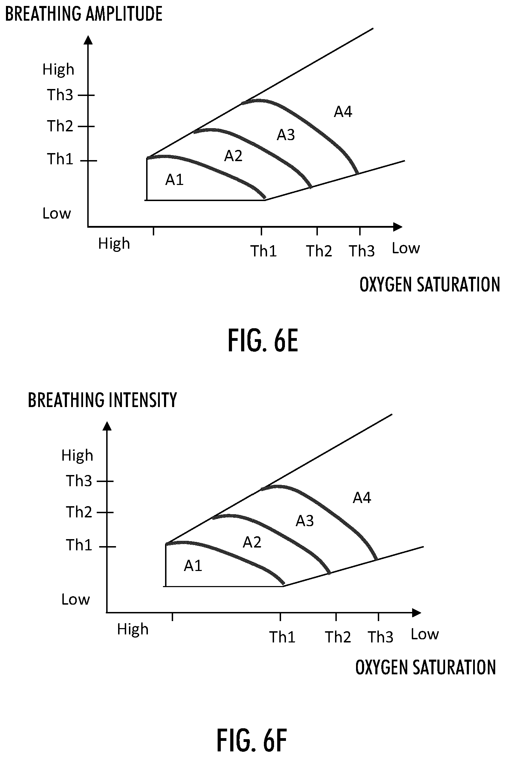

Fig 6E illustrates an example where parameter pairs are oxygen saturation level on x-axis and breathing amplitude on y-axis.

Fig 6F illustrates an example where parameter pairs are oxygen saturation level on x-axis and breathing intensity on y-axis.

It is also possible to combine different parameters, such as combined breathing effort parameter (combining breathing amplitude (amount of modulation inhale/exhale), breathing frequency (number breathing events per minute), breathing volume (litres/minute) and/or breathing intensity (breathing flow, breathing speed /duration of inhale/exhale).

The probability index can be calculated using the defined probability factors defined. In an embodiment, the probability index can be counted as a sum of the probability factors. In an embodiment, it is also possible to apply different weights to the factors selected. For example, the parameter pair heart rate - breathing frequency pair may be weighted by multiplying the factor of the pair by 1.5. The weighting may be done based on the patient's personal data or background information. For example, it is known that breathing irregularity is more obviously related to pulmonary edema in older people, so the breathing irregularity weight may be to 2.0 for older than 50 year old patients. and to 2.5 at older than 70 years ones. It is also known that different diseases and patient's conditions may increase a risk for pulmonary edema, so these factors can be taken into consideration using a weight factor. For example, if a patient is a smoker the weight can be set 1.5 or 2, or if a patient is a man the weight can be 1.25.

In an embodiment, when the probability index is a sum of probability factors, the sum can be made relative by dividing it by the number of contributing probability factors. The relative probability sum can be used as the measure of probability index. It can be scaled to be more representative for indication and alarming purposes.

For indication and alarming purposes thresholds maybe set for the probability index. The threshold may be set to be constant or varying depending on the patient's condition and other data. It can vary due to the patients age, gender, etc. In a non-limiting numerical example, the probability index threshold may be set to 0.75. It would mean that if four parameter pairs are used, then three of them are at least in the second area (A2), or one parameter pair is in the area of A3 and two are in the areas of A2 and one is in the normal area of A1, for example.

Thus, the probability index may be compared to a threshold and if the threshold is exceeded, an indication may be given. In an embodiment, the nature of the indication may depend upon how much the threshold is exceeded.

Liquid infusion monitors, counter devices or drip counters are used in medicine in connection with intravenous infusion therapy. There are also liquid infusion feeders or pumps which may be automatic or semiautomatic. They also have means for infusion monitoring. By means of an infusion monitor, it is made sure that a patient receives the desired amount of infusion liquid within the desired time.

Dripping rate of infusion liquid refers to the rate at which infusion liquid is administered for the patient. Conventionally, the dripping rate is indicated as the measured number of drips per minute, but the dripping rate may also be indicated as millilitres per hour, if the volume of one drip is known in advance and if the aforementioned minute-specific number of drips has been determined.

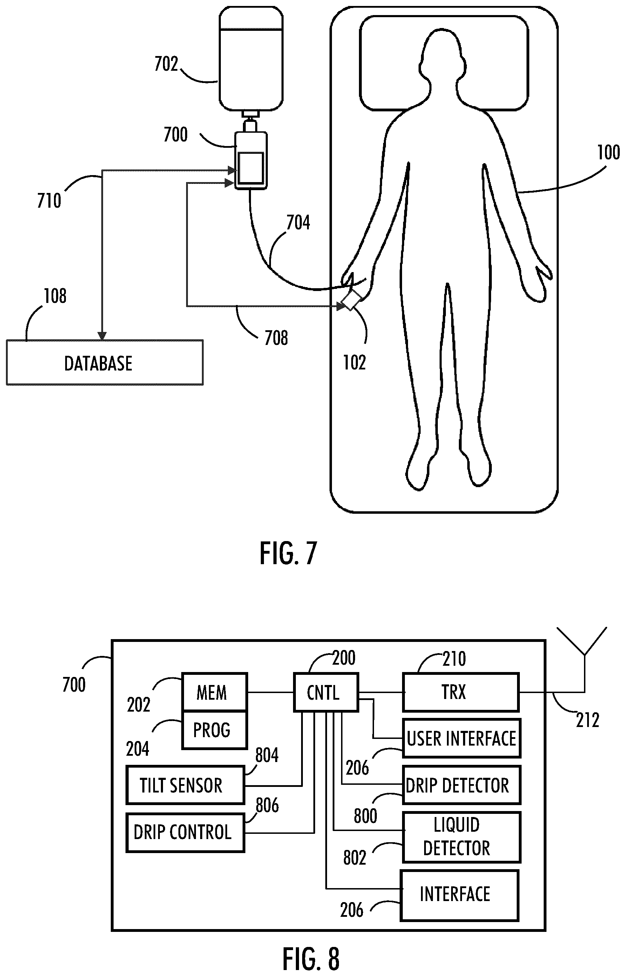

Figs. 7 and 8 illustrates an example of an apparatus of Fig.1 to which a liquid infusion monitor 700 has been added. Fig. 7 shows a liquid infusion monitor 700, to which a container 702 of infusion liquid is attached. The liquid infusion monitor is configured to administer 704 infusion liquid to a patient 100 so that the patient acquires a desired amount of infusion liquid within the desired time. The liquid infusion monitor typically controls the infusion rate or dripping rate of infusion liquid. The dripping rate refers to the rate at which infusion liquid is administered for the patient.

The liquid infusion monitor 700 is operationally connected 708 to a sensor 102. The sensor is configured to perform non-invasive optical measurements on a patient utilising at least two wavelengths.

A liquid infusion monitor typically comprises a drip detector which is configured to measure the dripping rate. The drip detector may be realized, for example with an optical detector, such as an LED (light emitting diode) light source and a phototransistor as its pair. Modern liquid infusion monitors are such that the device may show on a display the number of the drips in a given time, or the dripping rate such as drops/minute or drops/hour, dropped volume counted or the drops counted. In addition, a liquid infusion monitor may feature adjustable alarm thresholds by means of which the device may provide an alarm for the nursing staff if the dripping rate of the infusion liquid is not at the desired level or if a preset maximum amount of infusion liquid has been administered, that is, dripped.

In an embodiment, machine learning algorithm may be utilised in determining the probability of lung conditions. The machine learning algorithm may be trained using known measurement data, such as hospital database and measurement data. Then as measurement values are determined, they can be input to the machine learning algorithm which may then provide an estimate of a probability index for lung condition. New measurement data with determined probability index values and health care person inputs can be combined to create new use cases for analysis and training material. Further different other data inputs such as camera or video monitoring of the patient and care of patients as well other measurement data such earlier x-ray images, blood analysis or other laboratory results can be used to enhance the measured data.

Fig. 8 illustrates an embodiment. The figure illustrates a simplified example of a liquid infusion monitor 700.

It should be understood that the apparatus is depicted herein as an example illustrating some embodiments. It is apparent to a person skilled in the art that the apparatus may also comprise other functions and/or structures and not all described functions and structures are required. Although the apparatus has been depicted as one entity, different modules and memory may be implemented in one or more physical or logical entities.

Compared to the apparatus of Fig.1, in this embodiment the liquid infusion monitor acts also as the controller 104.

In an embodiment, the liquid infusion monitor 700 of the example includes a control circuitry 200 configured to control at least part of the operation of the apparatus. The control circuitry may be realized with at least one processor, for example.

The liquid infusion monitor may comprise a memory 202 for storing data. Furthermore, the memory may store software 204 executable by the control circuitry or processor 200. The memory may be integrated in the control circuitry.

In an embodiment, the liquid infusion monitor may comprise a detector 802 configured to determine presence of infusion liquid to be administered. The detector may be a mechanical or optical switch connected to the control circuitry or processor 200, for example.

In an embodiment, the liquid infusion monitor may comprise a drip control unit 806, which controls the amount of liquid to be administered for the patient. The liquid infusion monitor further comprises a drip detector 800 configured to measure the dripping rate. The liquid infusion monitor may further comprise a tilt sensor 804 which may determine if the device is not in upright position, in which case the operation of the liquid infusion monitor may be incorrect.

The liquid infusion monitor may comprise a transceiver 210 which may be connected to an antenna 212. The transceiver 210 may be a wireless transceiver utilizing known radio technology. An example of suitable radio technology is Bluetooth^{®}. Example of other possible technologies are wireless local area network (WLAN or WiFi), worldwide interoperability for microwave access (WiMAX), ZigBee^{®} and various cellular communication technologies well known in the art. In an embodiment, the transceiver is a wired transceiver connected with a wired connection to a suitable network such as the Internet.

In an embodiment, the liquid infusion monitor 104 may be configured to communicate 710, utilising the transceiver 210, with a hospital database 108 and/or an external monitoring device, for example. The data base may comprise various information, such as infusion liquid data (data of different liquids used in the hospitals), care plans and medical dosing data of patients of the hospitals and use condition data of infusion monitors. The use condition may comprise data related to the environment of drip counters such as the department of a hospital, the room number, bed number where the drip counter is used, for example.

The liquid infusion monitor may comprise a user interface 206, which may comprise a display, a keypad and a speaker, for example. The display may be touch sensitive in which case a keypad or keyboard is not necessarily required. In an embodiment, the user interface is at least partly realized in a remote monitoring apparatus connected to the liquid infusion monitor via the transceiver in a wired or wireless manner.

In an embodiment, the apparatus of Fig. 1 or Fig. 7 is configured to obtain information on hydration level of the measurement subject or patient 100.

The hydration level monitoring may be based on the AC and DC components of the oxygen saturation result. The AC pulse amplitude is related to blood volume and blood volume correlates with hydration level.

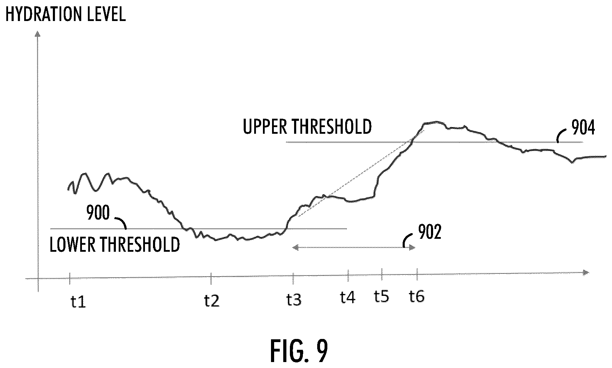

Fig. 9 illustrates an example of monitoring and utilising hydration level. The hydration level of a patient is monitored utilising apparatus of Fig. 7.

In this example, the measurement is started at time instant t1. At time t2 it is detected that hydration level is too low, being below the lower threshold 900. Thus, an indication may be given. At time instant t3 personnel may start infusion 902 using the liquid infusion monitor. Between time instants t3 and t4 infusion is going well and the hydration level is increasing.

At time instant t4 the apparatus detects that hydration is decreasing and it may give an indication. At time instant t5 hydration is going again well. At time instant t6 hydration has reached upper threshold 904, and the apparatus gives an indication. Personnel may stop giving infusion 902.

In an embodiment, the apparatus is configured to compare the rate of infusion given by the liquid infusion monitor to a given third upper threshold and generate an indication if the rate of infusion is greater than given third upper threshold and if the probability index is over a given threshold. In an embodiment, the indication may be an alarm to stop giving infusion 902.

In an embodiment, the apparatus is configured to receive the probability index. The apparatus will compare the probability index to the threshold. If the probability index is higher than the threshold set, it will indicate the status of probability index, and indicate/alarm the user/health care person that infusion must be stopped. In the case that the apparatus has also the means to stop the infusion it can stop the infusion in the case of the probability index exceeds the threshold set.

The steps and related functions described in the above and attached figures are in no absolute chronological order, and some of the steps may be performed simultaneously or in an order differing from the given one. Other functions can also be executed between the steps or within the steps. Some of the steps can also be left out or replaced with a corresponding step.

The apparatuses or controllers able to perform the above-described steps may be implemented as an electronic digital computer, which may comprise a working memory (RAM), a central processing unit (CPU), and a system clock. The CPU may comprise a set of registers, an arithmetic logic unit, and a controller. The controller is controlled by a sequence of program instructions transferred to the CPU from the RAM. The controller may contain a number of microinstructions for basic operations. The implementation of microinstructions may vary depending on the CPU design. The program instructions may be coded by a programming language, which may be a high-level programming language, such as C, Java, etc., or a low-level programming language, such as a machine language, or an assembler. The electronic digital computer may also have an operating system, which may provide system services to a computer program written with the program instructions.

As used in this application, the term 'circuitry' refers to all of the following: (a) hardware-only circuit implementations, such as implementations in only analog and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present.

This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network device, or another network device.

The computer program may be in source code form, object code form, or in some intermediate form, and it may be stored in some sort of carrier, which may be any entity or device capable of carrying the program. Such carriers include a record medium, computer memory, read-only memory, and a software distribution package, for example. Depending on the processing power needed, the computer program may be executed in a single electronic digital computer or it may be distributed amongst a number of computers.

The apparatus may also be implemented as one or more integrated circuits, such as application-specific integrated circuits ASIC. Other hardware embodiments are also feasible, such as a circuit built of separate logic components. A hybrid of these different implementations is also feasible. When selecting the method of implementation, a person skilled in the art will consider the requirements set for the size and power consumption of the apparatus, the necessary processing capacity, production costs, and production volumes, for example.

It will be obvious to a person skilled in the art that, as the technology advances, the inventive concept can be implemented in various ways. The invention and its embodiments are not limited to the examples described above but may vary within the scope of the claims.

## Claims

1. An apparatus for monitoring lung condition, the apparatus comprising
at least one sensor (102);
at least one processor (200);
at least one memory (202) including computer program code (204);
the at least one memory and the computer program code configured to, with the at least one processor, cause the apparatus at least to perform:
perform (300), utilising the at least one sensor, non-invasive optical measurements on a measurement subject utilising at least two wavelengths;
determine (302), based on the optical measurements, oxygen saturation, heart rate and/or heart rate variations of the measurement subject;
determine (304), based on heart rate and/or heart rate variations, breathing effort of the measurement subject;
detect (306) a change in breathing effort;
detect (308) a change in oxygen saturation level;
determine (310), based on the detected changes, a probability index for lung problem;
compare (312) the probability index to a given threshold;
generate (314) an indication if the probability index is over a given threshold.

2. The apparatus of claim 1, wherein the lung condition is pulmonary edema.

3. The apparatus of claim 1, the at least one memory and the computer program code configured to, with the at least one processor, cause the device further to perform:
compare the breathing effort to a given one or more thresholds;
compare the oxygen saturation level to a given first lower threshold;
utilise results of the comparisons when determining the probability index.

4. The apparatus of claim 3, the at least one memory and the computer program code configured to, with the at least one processor, cause the device further to perform:
compare the breathing effort to a given first upper threshold and given second lower threshold;
utilise results of the comparisons when determining the probability index.

5. The apparatus of claim 3 or 4, the at least one memory and the computer program code configured to, with the at least one processor, cause the device further to perform:
utilise threshold comparisons of two of following when determining the probability index: the breathing effort, the oxygen saturation level, heart rate, heart rate variations.

6. The apparatus of claim 3, 4 or 5, the at least one memory and the computer program code configured to, with the at least one processor, cause the device further to perform:
increase the probability index when a monitored value exceeds an upper threshold or falls below a lower threshold.

7. The apparatus of any preceding claim 3 to 6, the at least one memory and the computer program code configured to, with the at least one processor, cause the device further to perform:
decrease the probability index when a monitored value falls below an upper threshold or exceeds a lower threshold.

8. The apparatus of claim 4 and 5, the at least one memory and the computer program code configured to, with the at least one processor, cause the device further to perform:
increase the probability index in relation to the amount a monitored value exceeds an upper threshold or falls below a lower threshold.

9. The apparatus of any preceding claim, wherein one of the at least two wavelengths is between 620 to 730 nm and another of the at least two wavelengths is between 810 to 960 nm.

10. The apparatus of any preceding claim, the at least one memory and the computer program code configured to, with the at least one processor, cause the device further to perform:
obtain information on hydration level of the measurement subject based on pulsatile component and baseline component of the oxygen saturation result.

11. The apparatus of claim 10, further comprising
a liquid infusion monitor for monitoring infusion given to the measurement subject,
a second sensor for determining the rate of infusion administered by the liquid infusion monitor;
the at least one memory and the computer program code configured to, with the at least one processor, cause the apparatus further to perform:
determine the amount of infusion administered in a given time period,
take the hydration level and the determined amount of infusion into account when determining the probability index.

12. The apparatus of claim 11, the at least one memory and the computer program code configured to, with the at least one processor, cause the device further to perform:
compare the rate of infusion given by the liquid infusion monitor to a given third upper threshold;
generate an indication if the rate of infusion is greater than given third upper threshold and if the probability index is over a given threshold.

13. The apparatus of claim 11, the at least one memory and the computer program code configured to, with the at least one processor, cause the device further to perform:
generate an indication to stop administration of infusion based on the probability index.

14. The apparatus of any preceding claim, wherein the at least one sensor, the at least one processor and the at least one memory are operationally connected to each other.

15. The apparatus of claim 11, wherein the liquid infusion monitor is operationally connected to the at least one sensor, the at least one processor and the at least one memory.

16. The apparatus of any preceding claim, further comprising a transceiver, the at least one memory and the computer program code configured to, with the at least one processor, cause the apparatus further to control the transceiver to communicate with a hospital database and/or an external monitoring device.

17. The apparatus of any preceding claim, the at least one memory and the computer program code configured to, with the at least one processor, cause the device further to perform:
receive information on age, sex, medication of the measurement subject and take received information into account when determining the probability index.

18. The apparatus of any preceding claim, the at least one memory and the computer program code configured to, with the at least one processor, cause the device further to perform:
provide the determined values and detected changes as input to a machine learning algorithm, which has been trained using known values, and
obtain a probability index from the output of the algorithm.

19. A method for monitoring lung condition, the method comprising performing (300), utilising at least one sensor, non-invasive optical measurements on a measurement subject utilising at least two wavelengths;
determining (302), based on the optical measurements, oxygen saturation, heart rate and/or heart rate variations of the measurement subject;
determining (304), based on heart rate and/or heart rate variations, breathing effort of the measurement subject;
detecting (306) a change in breathing effort;
detecting (308) a change in oxygen saturation level;
determining (310), based on the detected changes, a probability index for lung problem;
comparing (312) the probability index to a given threshold;
generating (314) an indication if the probability index is over a given threshold.

20. A computer program comprising instructions for causing an apparatus execute the steps of
controlling a sensor to perform, utilising at least one sensor, non-invasive optical measurements on a measurement subject utilising at least two wavelengths;
determining, based on the optical measurements, oxygen saturation, heart rate and/or heart rate variations of the measurement subject;
determining, based on heart rate and/or heart rate variations, breathing effort of the measurement subject;
detecting a change in breathing effort;
detecting a change in oxygen saturation level;
determining, based on the detected changes, a probability index for lung problem;
comparing the probability index to a given threshold;
generating an indication if the probability index is over a given threshold.
